# EUROPEAN PATENT APPLICATION

(11) **EP 2 857 059 A1**
(43) Date of publication of application: **08.04.2015**
(21) Application number: 13797655.1
(22) Date of filing: 30.05.2013
(51) Int. Cl.: A61M 25/06

(54) **INDWELLING NEEDLE DEVICE**

(30) Priority: 31.05.2012 JP 2012125045
(71) Applicant: JMS Co., Ltd., Hiroshima-shi Hiroshima 730-8652 (JP)
(72) Inventor: FUJII, Ryoji, Hiroshima-shi, Hiroshima 730-8652 (JP); UEMATSU, Raita, Hiroshima-shi, Hiroshima 730-8652 (JP)
(74) Representative: Piésold, Alexander James
(86) International application number: PCT/JP2013/065080
(87) International publication number: WO 2013/180232

(57) **Abstract**

An outer unit (101) includes a shield (20) that has an inner cavity (24) and a soft outer needle (30) that is fixed to a front end of the shield. An inner unit (102) includes an inner hub (40) that is disposed within the inner cavity of the shield, a hard inner needle (50) that is fixed to a front end of the inner hub, and a tube (60) that is connected to a rear end of the inner hub. The inner unit is displaceable relative to the outer unit from an initial position at which the inner needle penetrates the outer needle and protrudes from a leading end of the outer needle to a retracted position at which the inner needle is housed within the inner cavity of the shield. The inner unit located at the initial position is fastened to the outer unit so that the inner unit is prevented from moving toward the retracted position due to a reaction force that is exerted on the inner needle during insertion with the inner unit being disposed at the initial position.

## Description

### Technical Field

The present invention relates to an indwelling needle device that includes a soft outer needle and a hard inner needle, and that is configured such that it can be inserted into a patient in a state in which a leading end of the inner needle protrudes from a leading end of the outer needle and then the inner needle can be retracted from the outer needle.

### Background Art

Indwelling needle devices are widely used for such treatments as infusion, blood transfusion, and extracorporeal blood circulation. In such treatments, leaving a metal needle inside a blood vessel may injure the blood vessel. Thus, indwelling needle devices are known that include a soft outer needle and a hard inner needle. The outer needle and the inner needle are inserted into a blood vessel of a patient in a state in which a leading end of the inner needle protrudes from a leading end of the outer needle, and then the inner needle is retracted from the outer needle, so that only the outer needle remains in the patient. The possibility that the remaining soft outer needle will injure the blood vessel of the patient is low.

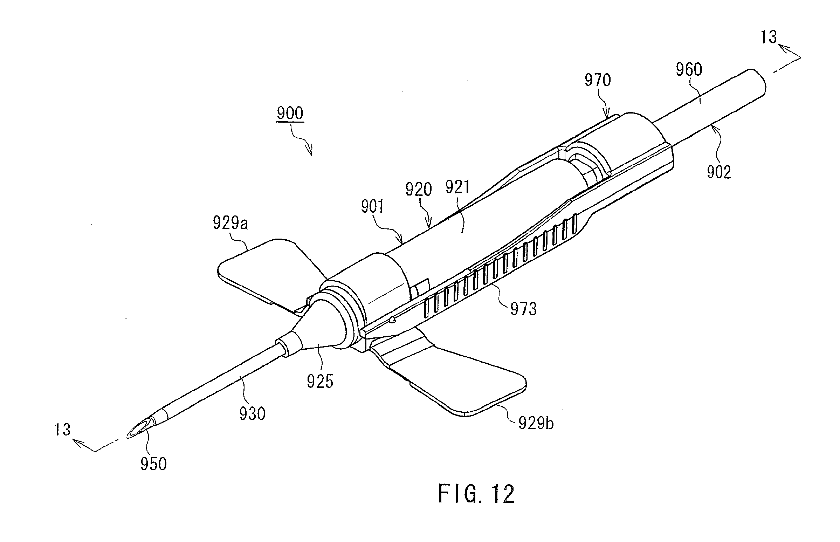

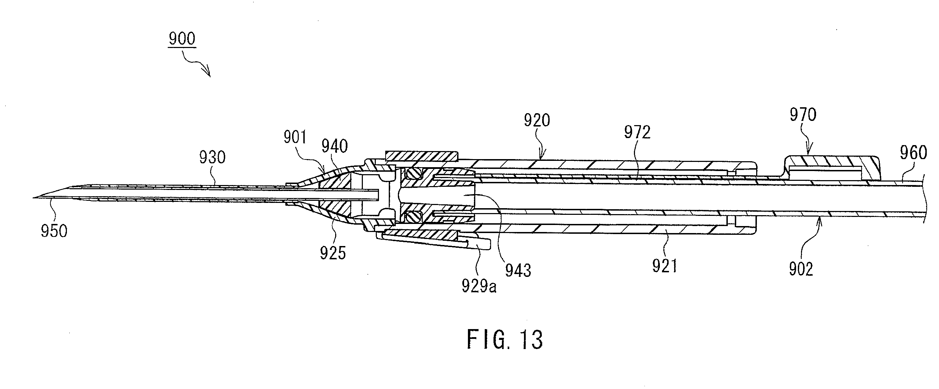

FIG. 12 is a perspective view of an example of such a conventional indwelling needle device 900 (see Patent Document 1, for example) as viewed from above. FIG. 13 is a cross-sectional view of the conventional indwelling needle device 900 taken along a vertical plane containing line 13-13 in FIG. 12 and viewed in the direction of arrows 13. For the sake of convenience of description, a side that is inserted into the patient (the left side in FIGS. 12 and 13) is referred to as a "front side", and a side that is opposite from this side is referred to as a "rear side".

The indwelling needle device 900 includes a shield 920 configured by an approximately cylindrical shield tube 921 and an outer hub 925 that is fixed to an end (front end) of the shield tube 921. A soft outer needle 930 is fixed to a front end of the outer hub 925. A pair of wings 929a and 929b are provided on an outer circumferential face of the shield tube 921 in the vicinity of its outer hub 925 side end. The wings 929a and 929b are flexible, and can be bent up and down.

An inner hub 940 is inserted in an inner cavity of the shield 920. A hard inner needle 950 made of metal is fixed to a front end of the inner hub 940, and one end of a flexible tube 960 is connected to a rear end of the inner hub 940. The inner needle 950 and the tube 960 are in communication with each other via a longitudinal penetration path 943 that penetrates the inner hub 940 in a front-rear direction.

The shield tube 921, the outer hub 925, the outer needle 930, and the wings 929a and 929b constitute an outer unit 901 of the indwelling needle device 900. On the other hand, the inner hub 940, the inner needle 950, and the tube 960 constitute an inner unit 902 of the indwelling needle device 900. The inner unit 902 is inserted in the outer unit 901 so as to be movable in a longitudinal direction (i.e., front-rear direction) of the shield 920.

In FIGS. 12 and 13, the inner hub 940 is located on the front end side of the inner cavity of the shield 920, the inner needle 950 that is held by the inner hub 940 penetrates the outer needle 930, and the leading end of the inner needle 950 protrudes to the outside from the leading end of the outer needle 930. This position of the inner unit 902 relative to the outer unit 901 is referred to as an "initial position".

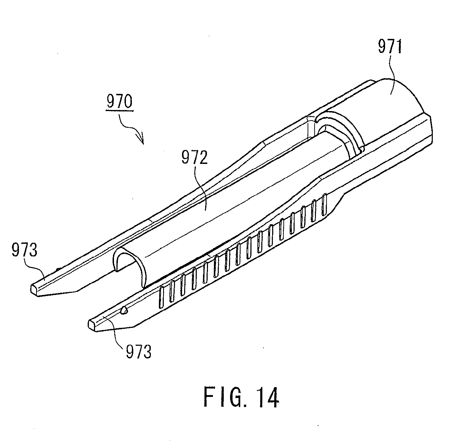

In order to keep the inner unit 902 at the initial position, a stopper 970 is used. FIG. 14 is a perspective view of the stopper 970. An approximately semi-cylindrical insertion portion 972 and a pair of fixing portions 973 extend from an approximately semi-cylindrical base portion 971. The insertion portion 972 is disposed between the pair of fixing portions 973, and these portions are parallel to one another.

As shown in FIG. 13, the insertion portion 972 of the stopper 970 is inserted from the rear end of the shield tube 921. When a leading end of the insertion portion 972 abuts the rear end of the inner hub 940 and pushes the inner hub 940 toward the front side, the inner unit 902 can be disposed at the initial position.

The indwelling needle device 900 is used as follows.

First, the inner needle 950 and the outer needle 930 are inserted into a blood vessel of the patient in a state in which the inner unit 902 is kept at the initial position (insertion operation). It is necessary to prevent the inner needle 950 from being pushed back into the outer needle 930 due to a reaction force that is exerted on the inner needle 950 by the patient during insertion. For this reason, it is necessary to perform the insertion operation while holding the stopper 970. Thus, the stopper 970 and the inner unit 902 are prevented from being displaced relative to the outer unit 901, and the inner unit 902 is maintained at the initial position.

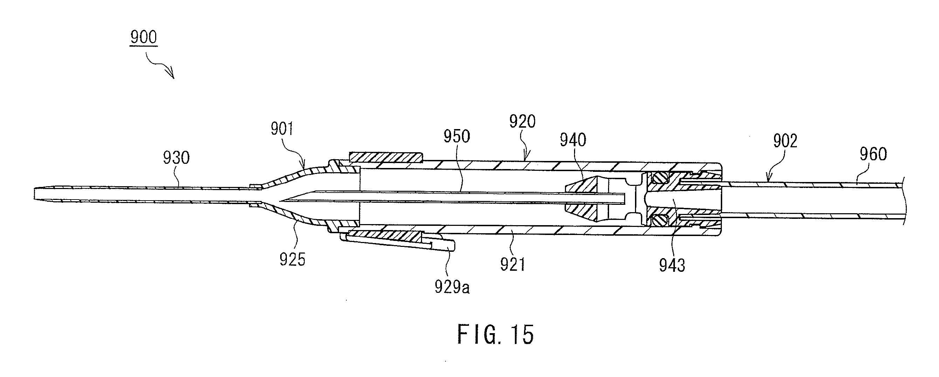

After the insertion of the inner needle 950 and the outer needle 930 into the blood vessel of the patient, the stopper 970 is pulled out of the shield 920, and then the tube 960 is pulled from the shield 920 (retraction operation). The stopper 970 may be pulled out of the shield 920 at the same time that the tube 960 is pulled. When the tube 960 is pulled, the inner unit 902 is moved toward the rear side relative to the outer unit 901, and the inner needle 950 is housed within the shield 920 as shown in FIG. 15. The position of the inner unit 902 relative to the outer unit 901 shown in FIG. 15 is referred to as a "retracted position". In this state, the indwelling needle device 900 is fixed to the patient using an adhesive tape or the like. The indwelling needle device 900 remains in the patient in a state in which only the soft outer needle 930 is inserted in the patient.

### Citation List

### Patent Document

Patent Document 1: JP 2011-251081A

### Disclosure of Invention

### Problem to be Solved by the Invention

According to the conventional indwelling needle device 900, the stopper 970 is used solely to maintain the inner unit 902 at the initial position during the insertion operation that inserts the inner needle 950 and the outer needle 930 into the patient. The stopper 970 is pulled out of the shield 920 prior to or simultaneously with the following retraction operation that moves the inner unit 902 to the retracted position.

However, there is a risk that an operating mistake of forgetting to pull the tube 960 may be made under an illusion that the inner unit 902 has been moved to the retracted position by merely pulling the stopper 970 out of the shield 920. As a result, the indwelling needle device 900 remains in the patient in a state in which the inner needle 950 protrudes from the leading end of the outer needle 930, and thus there is a possibility that the leading end of the hard inner needle 950 may injure the blood vessel of the patient.

Moreover, provision of the stopper 970 increases the number of components that constitute the indwelling needle device 900, resulting in an increase in the cost of the indwelling needle device 900.

Furthermore, there is a possibility that the stopper 970 inserted in the shield 920 may come out of the shield 920 due to vibrations or the like during transportation of the indwelling needle device 900.

The present invention has been made to solve the above-described problems with a conventional indwelling needle device, and it is an object thereof to provide an indwelling needle device in which an inner unit can be maintained at an initial position without usage of a stopper during an insertion operation and thus the stopper is omitted.

### Means for Solving Problem

An indwelling needle device according to the present invention includes an outer unit including a shield that has an inner cavity and a soft outer needle that is fixed to a front end of the shield, and an inner unit including an inner hub that is disposed within the inner cavity of the shield, a hard inner needle that is fixed to a front end of the inner hub, and a tube that is connected to a rear end of the inner hub. The inner unit is displaceable relative to the outer unit from an initial position at which the inner needle penetrates the outer needle and protrudes from a leading end of the outer needle to a retracted position at which the inner needle is housed within the inner cavity of the shield. The inner unit located at the initial position is fastened to the outer unit so that the inner unit is prevented from moving toward the retracted position due to a reaction force that is exerted on the inner needle during insertion with the inner unit being disposed at the initial position.

### Effects of the Invention

According to the present invention, the inner unit located at the initial position is fastened to the outer unit, and thus the inner unit is prevented from moving toward the retracted position due to a reaction force that is exerted on the inner needle during insertion with the inner unit being disposed at the initial position. Therefore, a stopper that is necessary in a conventional indwelling needle device can be omitted.

### Brief Description of Drawings

[FIG. 1] FIG. 1 is a perspective view of an indwelling needle device according to Embodiment 1 of the present invention as viewed from above, with an inner unit being at the initial position.
[FIG. 2] FIG. 2 is a cross-sectional view of the indwelling needle device according to Embodiment 1 of the present invention taken along a vertical plane containing line 2-2 in FIG. 1 and viewed in the direction of arrows 2.
[FIG. 3] FIG. 3A is a perspective view of an inner hub used in the indwelling needle device according to Embodiment 1 of the present invention, FIG. 3B is a cross-sectional view of the inner hub taken along a plane containing line 3B-3B in FIG. 3A and viewed in the direction of arrows 3B, and FIG. 3C is a cross-sectional view of the inner hub taken along a plane containing line 3C-3C in FIG. 3A and viewed in the direction of arrows 3C.
[FIG. 4] FIG. 4 is a perspective view of the indwelling needle device according to Embodiment 1 of the present invention as viewed from above, with the inner unit being at the retracted position.
[FIG. 5] FIG. 5 is a cross-sectional view of the indwelling needle device according to Embodiment 1 of the present invention taken along a vertical plane containing line 5-5 in FIG. 4 and viewed in the direction of arrows 5.
[FIG. 6] FIG. 6 is a cross-sectional view showing leading ends of an inner needle and an outer needle, and the vicinity thereof, of the indwelling needle device according to Embodiment 1 of the present invention when the inner unit is at the initial position.
[FIG. 7] FIG. 7 is a cross-sectional view showing an outer hub and an inner hub, and the vicinity thereof, of an indwelling needle device according to Embodiment 2 of the present invention when an inner unit is at the initial position.
[FIG. 8] FIG. 8 is a cross-sectional view showing an inner hub, and the vicinity thereof, of an indwelling needle device according to Embodiment 3 of the present invention when an inner unit is at the initial position.
[FIG. 9] FIG. 9 is a cross-sectional view showing an inner hub, and the vicinity thereof, of an indwelling needle device according to Embodiment 4 of the present invention when an inner unit is at the initial position.
[FIG. 10] FIG. 10 is a cross-sectional view showing an inner hub, and the vicinity thereof, of an indwelling needle device according to Embodiment 5 of the present invention when an inner unit is at the initial position.
[FIG. 11] FIG. 11 is a cross-sectional view showing an outer hub, and the vicinity thereof, of an indwelling needle device according to Embodiment 6 of the present invention when an inner unit is at the initial position.
[FIG. 12] FIG. 12 is a perspective view of a conventional indwelling needle device as viewed from above, with an inner unit being at the initial position.
[FIG. 13] FIG. 13 is a cross-sectional view of the conventional indwelling needle device taken along a vertical plane containing line 13-13 in FIG. 12 and viewed in the direction of arrows 13.
[FIG. 14] FIG. 14 is a perspective view of a stopper used in the conventional indwelling needle device shown in FIG. 12.
[FIG. 15] FIG. 15 is a cross-sectional view of the conventional indwelling needle device shown in FIG. 12 taken along the same plane as in FIG. 13, with the inner unit being at the retracted position.

### Description of the Invention

An indwelling needle device of the present invention includes an outer unit including a shield that has an inner cavity and a soft outer needle that is fixed to a front end of the shield, and an inner unit including an inner hub that is disposed within the inner cavity of the shield, a hard inner needle that is fixed to a front end of the inner hub, and a tube that is connected to a rear end of the inner hub. The inner unit is displaceable relative to the outer unit from an initial position at which the inner needle penetrates the outer needle and protrudes from a leading end of the outer needle to a retracted position at which the inner needle is housed within the inner cavity of the shield. The inner unit located at the initial position is fastened to the outer unit so that the inner unit is prevented from moving toward the retracted position due to a reaction force that is exerted on the inner needle during insertion with the inner unit being disposed at the initial position. It should be understood that "fastening" between the inner unit and the outer unit as used in the present invention means that it is enough if displacement of the inner unit relative to the outer unit due to the reaction force exerted on the inner needle during insertion can be prevented and does not mean that movement of the inner unit relative to the outer unit is permanently restricted.

The above-described indwelling needle device of the present invention may be configured such that when the inner unit is at the initial position, the inner needle is fastened to the outer needle.

The above-described indwelling needle device of the present invention may be configured such that when the inner unit is at the initial position, the inner hub is fastened to the shield.

The shield may include an outer hub that holds the outer needle and a tube-shaped shield tube, the outer hub being fixed to an end of the shield tube. In this case, when the inner unit is at the initial position, the inner hub may be fastened to the outer hub. Alternatively, when the inner unit is at the initial position, the inner hub may be fastened to the shield tube.

The above-described indwelling needle device of the present invention may be configured such that when the inner unit is at the initial position, an O-ring attached to the inner hub is fastened to the shield.

The shield may include an outer hub that holds the outer needle and a tube-shaped shield tube, the outer hub being fixed to an end of the shield tube. In this case, when the inner unit is at the initial position, the O-ring may be fastened to the shield tube. Alternatively, when the inner unit is at the initial position, the O-ring may be fastened to the outer hub.

The above-described indwelling needle device of the present invention may be configured such that when the inner unit is at the initial position, the inner needle is fastened to the shield.

The shield may include an outer hub that holds the outer needle and a tube-shaped shield tube, the outer hub being fixed to an end of the shield tube. In this case, when the inner unit is at the initial position, the inner needle may be fastened to the outer hub.

The above-described indwelling needle device of the present invention is preferably configured such that the fastening is caused by friction between the inner unit and the outer unit. With this configuration, the inner unit and the outer unit can be fastened to each other using a simple structure.

Hereinafter, the present invention will be described in detail while showing preferred embodiments thereof. However, it goes without saying that the present invention is not limited to the embodiments below. In the drawings that will be referred to in the following description, only main members of constituent members of the embodiments of the present invention that are necessary for the description of the present invention are shown in a simplified manner for the sake of convenience of description. Accordingly, the present invention may include optional constituent members that are not shown in the drawings below. Moreover, it should be understood that the dimensions of the members in the drawings below are not faithful representation of the dimensions of actual constituent members, dimensional ratios of those members, and the like.

### Embodiment 1

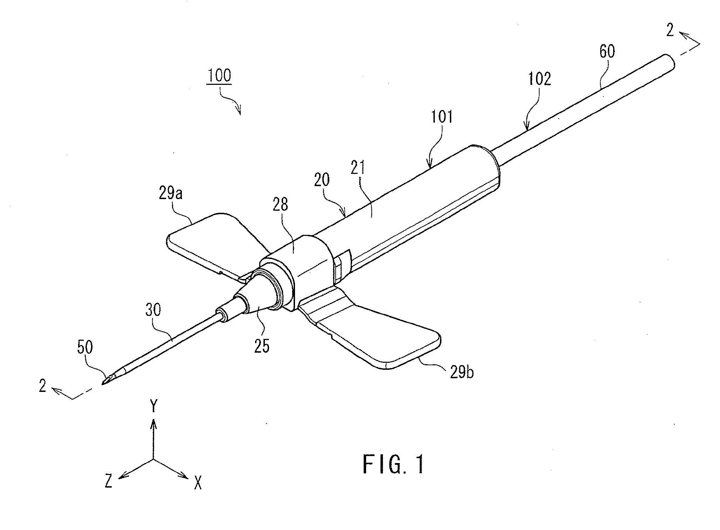

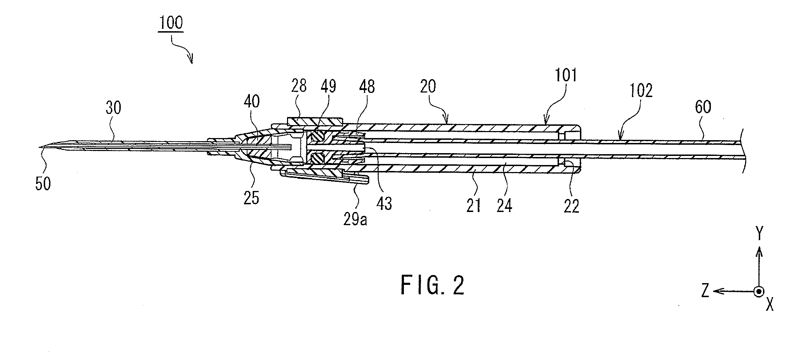

FIG. 1 is a perspective view, as viewed from above, of an indwelling needle device 100 according to Embodiment 1 of the present invention with an inner unit being at the initial position. For the sake of convenience of description, an orthogonal coordinate system is set in which the longitudinal direction of the indwelling needle device 100 is taken as a Z axis, and the horizontal axis and the vertical axis orthogonal to the Z axis are respectively taken as an X axis and a Y axis. Furthermore, a side in the direction of the Y axis arrow (i.e., the upper side in FIG. 1) is referred to as an "upper side", and a side that is opposite from this side is referred to as a "lower side". Note that the "horizontal direction" and the "vertical direction" do not refer to the orientations when actually using the indwelling needle device 100. Moreover, a side that is inserted into the patient (a side in the direction of the Z axis arrow, that is, the left side in FIG. 1) is referred to as a "front side", and a side that is opposite from this side is referred to as a "rear side". FIG. 2 is a cross-sectional view of the indwelling needle device 100 taken along a vertical plane (YZ plane) containing line 2-2 in FIG. 1 and viewed in the direction of arrows 2.

The indwelling needle device 100 includes a shield 20. The shield 20 has a shield tube 21 and an outer hub 25 that is fixed to an end (front end) of the shield tube 21. The shield tube 21 has an approximately cylindrical shape having a constant inner diameter. An engagement ridge 22 that is continuous in a circumferential direction is formed on an inner circumferential face of the shield tube 21 in the vicinity of an end (rear end) that is opposite from the outer hub 25. The outer hub 25 is approximately funnel-shaped, and a soft outer needle 30 is fixed to an end (front end) thereof that is opposite from the shield tube 21. The outer needle 30 has an approximately cylindrical shape. Although there is no particular limitation on the materials for the shield tube 21 and the outer hub 25, a hard material is preferable, and, for example, polycarbonate, polypropylene, and the like can be used. Preferably, the shield tube 21 and the outer hub 25 have transparency or translucency, so that fluid (medical fluid, blood, etc.) and an inner hub 40 inside an inner cavity 24 of the shield 20 can be seen therethrough. Although there is no particular limitation on the material for the outer needle 30, a soft material is preferable, and, for example, polypropylene, polyurethane elastomer, fluororesin such as polytetrafluoroethylene, and the like can be used. Preferably, the outer needle 30 has transparency or translucency, so that fluid (medical fluid, blood, etc.) and an inner needle 50 inside its inner cavity can be seen therethrough. It should be noted that the outer hub 25 and the outer needle 30 may also be integrally formed using the soft material described above.

Reference numerals 29a and 29b indicate wings that extend approximately parallel to the X axis. The wings 29a and 29b are provided on a fixing member 28 having an approximately cylindrical shape. The wings 29a and 29b are installed on the shield 20 by externally fitting the fixing member 28 to the outer circumferential face of the shield tube 21 in the vicinity of its outer hub 25 side end. Although there is no particular limitation on the material for the wings 29a and 29b, a soft material is preferable, and, for example, polypropylene, vinyl chloride, polyethylene, olefin or polystyrene thermoplastic elastomer, and the like can be used. It should be noted that the wings 29a and 29b may also be integrally molded with the shield 20.

The inner hub 40 is inserted in the inner cavity 24 of the shield 20 so as to be movable in the longitudinal direction (i.e., Z axis direction) of the shield 20. The hard inner needle 50 made of metal is fixed to a front end of the inner hub 40. The inner needle 50 has an approximately cylindrical shape, and the leading end thereof is processed to be sharp. One end of a flexible tube 60 is connected to a rear end of the inner hub 40. The other end of the tube 60 is connected to, for example, a drip infusion system for performing infusion. An O-ring 49 is installed on an outer circumferential face of the inner hub 40. The O-ring 49 is in close contact with the inner circumferential face of the shield tube 21 and prevents, in the inner cavity 24 of the shield 20, medical fluid or blood that is present on the outer needle 30 side with respect to the O-ring 49 from leaking to the tube 60 side with respect to the O-ring 49. Although there is no particular limitation on the material for the inner hub 40, a hard material is preferable, and, for example, polycarbonate, polypropylene, polyethylene, and the like can be used. Although there is no particular limitation on the material for the tube 60, a soft material is preferable, and, for example, a resin material such as vinyl chloride and the like can be used. Although there is no particular limitation on the material for the O-ring 49, a flexible and elastically deformable material is preferable, and, for example, polyisoprene rubber, silicone rubber, thermoplastic elastomer, and the like can be used.

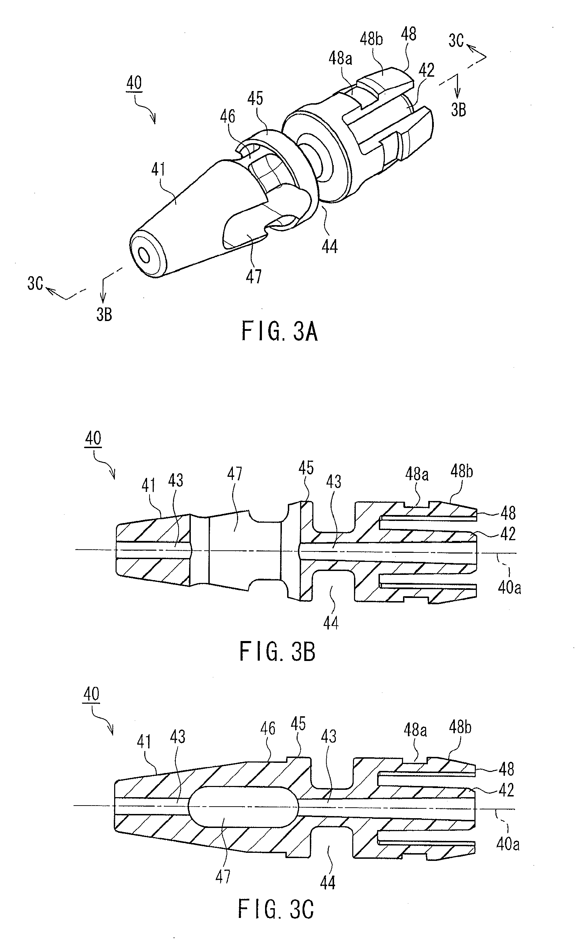

FIG. 3A is a perspective view of the inner hub 40, FIG. 3B is a cross-sectional view of the inner hub 40 taken along a plane containing line 3B-3B in FIG. 3A and viewed in the direction of arrows 3B, and FIG. 3C is a cross-sectional view of the inner hub 40 taken along a plane containing line 3C-3C in FIG. 3A and viewed in the direction of arrows 3C. The cross-section shown in FIG. 3B and the cross-section shown in FIG. 3C are orthogonal to each other. The inner hub 40 has at its end (front end) a front portion 41 having a circular conical outer face, and has at its other end a rear portion 42 having a cylindrical outer face. A longitudinal penetration path 43 longitudinally penetrates the inner hub 40 and extends along a central axis 40a of the inner hub 40 from the front portion 41 to the rear portion 42. As shown in FIG. 2, the inner needle 50 is inserted into the longitudinal penetration path 43 from the front portion 41 side and held by the inner hub 40. The rear portion 42 is inserted into the tube 60, so that the inner hub 40 is connected to the tube 60. Thus, the inner needle 50 and the tube 60 are in communication with each other via the longitudinal penetration path 43 of the inner hub 40.

An annular groove 44 that is continuous in a circumferential direction is formed in the outer circumferential face of the inner hub 40 in a location between the front portion 41 and the rear portion 42. As shown in FIG. 2, the O-ring 49 is installed in the annular groove 44.

A large diameter portion 45 and a small diameter portion 46 are formed in that order from the annular groove 44 side, in the outer circumferential face of the inner hub 40 in respective locations between the annular groove 44 and the front portion 41. The small diameter portion 46 is adjacent to the front portion 41, and the outer diameter of the small diameter portion 46 is substantially the same as the largest diameter of the front portion 41 and is smaller than the outer diameter of the large diameter portion 45. A lateral penetration path 47 that laterally penetrates the front portion 41, the small diameter portion 46, and the large diameter portion 45 in their diameter direction (direction orthogonal to the central axis 40a) is formed in these portions. The lateral penetration path 47 intersects and is in communication with the longitudinal penetration path 43.

Four projecting elastic members 48 are arranged around the rear portion 42 at equiangular intervals about the central axis 40a of the inner hub 40. The elastic members 48 extend approximately parallel to the central axis 40a of the inner hub 40. A fitting groove 48a and a tapered surface 48b are formed on a face of each elastic member 48 that is opposite from the rear portion 42. The fitting groove 48a is a recess (groove) extending in the circumferential direction of the inner hub 40. The tapered surface 48b is adjacent to the fitting groove 48a on a side thereof that is closer to the free end of the elastic member 48, and constitutes part of a circular conical face having an outer diameter that is larger toward the fitting groove 48a.

The shield 20 as well as the outer needle 30, the wings 29a and 29b, and the fixing member 28 that are fixed to the shield 20 constitute an outer unit 101 of the indwelling needle device 100. On the other hand, the inner hub 40, the inner needle 50, and the tube 60 constitute an inner unit 102 of the indwelling needle device 100. The inner unit 102 is inserted in the outer unit 101 so as to be movable in the longitudinal direction (i.e., front-rear direction) of the shield 20.

In FIGS. 1 and 2, the inner hub 40 is located on the front end side of the inner cavity 24 of the shield 20, the inner needle 50 held by the inner hub 40 penetrates the outer needle 30, and a leading end of the inner needle 50 protrudes to the outside from the leading end of the outer needle 30. In the present invention, this position of the inner unit 102 relative to the outer unit 101 is referred to as an "initial position".

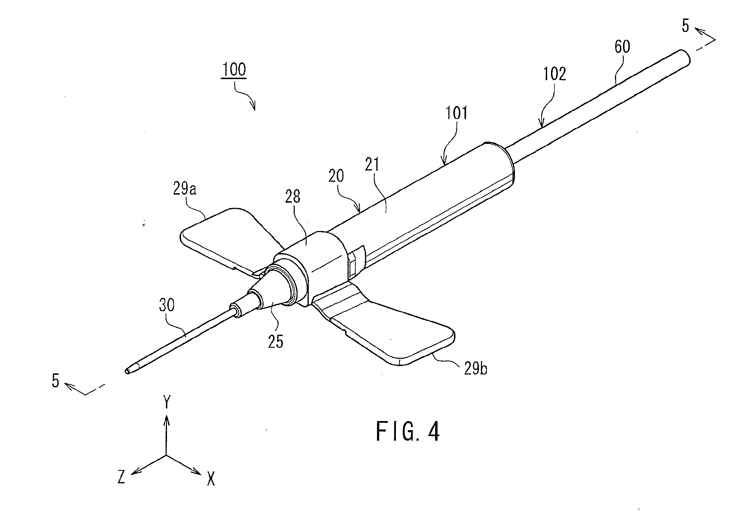

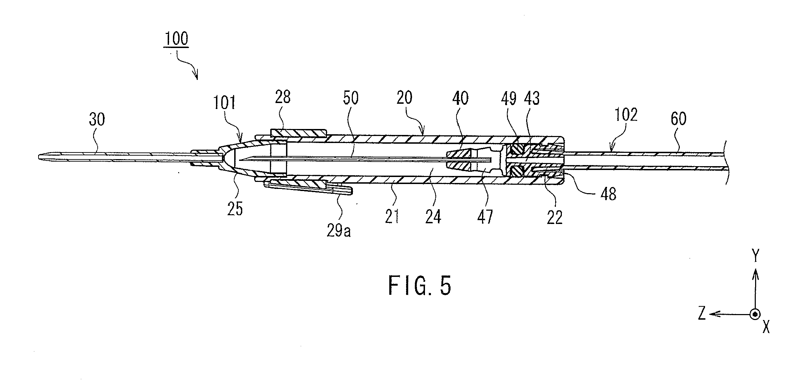

FIG. 4 is a perspective view, as viewed from above, of the indwelling needle device 100 with the inner unit 102 being at a retracted position. FIG. 5 is a cross-sectional view of the indwelling needle device 100 taken along a vertical plane (YZ plane) containing line 5-5 in FIG. 4 and viewed in the direction of arrows 5.

As shown in FIG. 5 when the inner unit 102 is at the "retracted position", the fitting grooves 48a (see FIGS. 3A, 3B, and 3C) of the inner hub 40 and the engagement ridge 22 of the shield tube 21 are fitted to each other. Moreover, the inner needle 50 held by the inner hub 40 has been pulled out of the outer needle 30 and is housed within the inner cavity 24 of the shield 20.

When compared with the initial position (see FIGS. 1 and 2), at the retracted position, the cross-sectional area of the flow channel within the outer needle 30 is increased by an amount corresponding to the cross-sectional area of the inner needle 50, and accordingly the flow rate of the medical fluid or blood is increased. Furthermore, at the retracted position, the flow channel from the outer needle 30 to the tube 60 includes two flow channels, that is, a first flow channel sequentially passing through the inner cavity of the inner needle 50 and the longitudinal penetration path 43 of the inner hub 40 and a second flow channel sequentially passing through a space between the inner face of the shield 20 and the respective outer faces of the inner needle 50 and the inner hub 40, the lateral penetration path 47 of the inner hub 40, and the longitudinal penetration path 43 of the inner hub 40, and accordingly the medical fluid or blood can flow at a high flow rate.

As described above, according to the indwelling needle device 100 of Embodiment 1, the inner unit 102 can move in the front-rear direction relative to the outer unit 101 from the initial position shown in FIGS. 1 and 2 to the retracted position shown in FIGS. 4 and 5.

During insertion of the indwelling needle device 100 into the patient, the inner unit 102 needs to be kept at the initial position. According to the indwelling needle device 100 of Embodiment 1, the inner unit 102 is fastened to the outer unit 101 so that the inner unit 102 located at the initial position is prevented from moving toward the retracted position.

Now, fastening of the inner unit 102 to the outer unit 101 will be described.

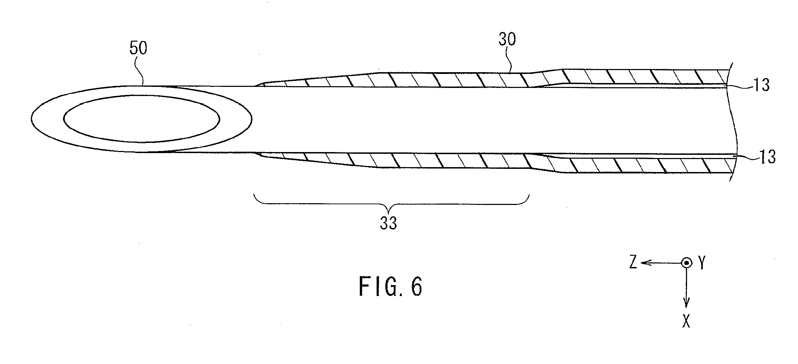

FIG. 6 is a cross-sectional view showing the leading ends of the outer needle 30 and the inner needle 50, and the vicinity thereof, when the inner unit 102 is at the initial position. In FIG. 6, only the outer needle 30 is shown in cross-section. As shown in FIG. 6, an outer circumferential face of the inner needle 50 is a cylindrical face having a constant outer diameter in the longitudinal direction (Z axis direction) of the inner needle 50. In contrast, the inner diameter of the outer needle 30 is not constant in the longitudinal direction of the outer needle 30. The inner diameter of the outer needle 30 is small in a region (small diameter region) 33 within a predetermined distance from the leading end of the outer needle 30 and is larger than this small inner diameter in a portion rearward of the small diameter region 33. Preferably, the inner diameter of the outer needle 30 in the small diameter region 33 is slightly smaller than the outer diameter of the inner needle 50. Accordingly, in the small diameter region 33, the outer needle 30 is in close contact with the inner needle 50 while being slightly elastically expanded in the circumferential direction and constricting the inner needle 50. On the other hand, in the portion rearward of the small diameter region 33, a gap 13 is formed between the outer needle 30 and the inner needle 50.

In this manner, when the inner unit 102 is at the initial position, the outer needle 30 is in close contact with the inner needle 50 in the small diameter region 33, and therefore the friction between the outer needle 30 and the inner needle 50 is large, causing the outer needle 30 and the inner needle 50 to be fastened to each other. Accordingly, to move the inner unit 102 located at the initial position to the retracted position, a large force that is enough to counteract the fastening force between the outer needle 30 and the inner needle 50 is required.

Now, a method for using the indwelling needle device 100 of Embodiment 1 having the above-described configuration and effects of this device will be described.

The indwelling needle device 100 of Embodiment 1 is delivered to a medical institution such as a hospital in a state in which the inner unit 102 is disposed at the initial position (FIGS. 1 and 2).

At the medical institution, an operator inserts the inner needle 50 and the outer needle 30 into a blood vessel of the patient in a state in which, as shown in FIGS. 1 and 2, the inner unit 102 is at the initial position and the inner needle 50 protrudes from the leading end of the outer needle 30 (insertion operation). It is possible to perform the insertion operation while gripping the outer unit 101 with one's fingers. At this time, a reaction force in a direction opposite to the insertion direction is exerted on the inner needle 50. This reaction force acts so as to move the inner unit 102 to the retracted position side relative to the outer unit 101. However, as described above, in the small diameter region 33 of the outer needle 30, the inner needle 50 is fastened to the outer needle 30, and thus the inner unit 102 does not move from the initial position relative to the outer unit 101.

Next, in a state in which the outer needle 30 is inserted in the patient, the inner unit 102 is retracted (retraction operation). For example, it is possible to pull the tube 60 from the outer unit 101 by gripping the tube 60 with one hand while holding the outer unit 101 with the other hand (e.g., the hand that is different from the hand by which the outer unit 101 is gripped during the insertion operation) so as to prevent the outer unit 101 from being displaced relative to the patient. To slide the inner needle 50 relative to the small diameter region 33 of the outer needle 30, it is necessary to apply a large tensile force to the tube 60. Afterward, when the inner needle 50 has come out of the small diameter region 33 of the outer needle 30, the tube 60 can be pulled with a relatively small tensile force. The inner unit 102 is moved rearward together with the tube 60.

The engagement ridge 22 is formed on the inner circumferential face of the shield tube 21 in the vicinity of its rear end. The inner hub 40 moves to the engagement ridge 22, and the tapered surfaces 48b (see FIGS. 3A, 3B, and 3C) formed on the respective outer faces of the elastic members 48 of the inner hub 40 slide on the engagement ridge 22. At this time, the elastic members 48 bend toward the rear portion 42 side. Then, when the tapered surfaces 48b have run over the engagement ridge 22, the elastic members 48 bend back to their original form, and the engagement ridge 22 is fitted into the fitting grooves 48a. In this manner, the inner unit 102 moves to the retracted position shown in FIGS. 4 and 5.

In this state, an adhesive tape is attached to the skin of the patient over the outer unit 101, and the indwelling needle device 100 is fixed to the patient. Only the outer needle 30 remains in the patient in a state in which it is inserted in the patient. When the inner unit 102 is at the retracted position, the hard inner needle 50 is not present in the flexible outer needle 30, and therefore, even if the position of the indwelling needle device 100 relative to the patient changes due to movement of the patient or the like, the outer needle 30 does not injure the blood vessel and the like of the patient.

When the necessary treatment has been finished, the adhesive tape that fixes the outer unit 101 to the patient is removed, and the outer needle 30 is withdrawn from the patient. Even when the tube 60 is pushed or pulled relative to the outer unit 101, the fitted state in which the fitting grooves 48a of the inner hub 40 and the engagement ridge 22 of the shield tube 21 are fitted to each other is not released. That is to say, the inner needle 50 cannot protrude again from the leading end of the outer needle 30, and the inner unit 102 cannot be pulled from the outer unit 101. Accordingly, accidental puncture with the hard inner needle 50 and accidental reuse of the used indwelling needle device 100 are prevented. The used indwelling needle device 100 will be discarded.

As described above, according to the indwelling needle device 100 of Embodiment 1, when the inner unit 102 is at the initial position, the inner needle 50 and the outer needle 30 are fastened to each other in the small diameter region 33 of the outer needle 30. Accordingly, even when a reaction force is exerted on the inner needle 50 during the insertion operation, the inner unit 102 does not move toward the retracted position and remains at the initial position. Therefore, the stopper 970 (FIG. 14), which is essential for the conventional indwelling needle device 900 (FIGS. 12 to 15) in order to prevent the inner unit 902 located at the initial position from moving toward the retracted position during the insertion operation, is no longer necessary in the indwelling needle device 100 of the Embodiment 1.

As described above, with the conventional indwelling needle device 900, there is a risk that an operating mistake may be made under an illusion that the inner unit 902 has been moved to the retracted position by pulling the stopper 970 out of the shield 920. In contrast, according to Embodiment 1, the stopper is not present. To move the inner unit 102 from the initial position to the retracted position, it is necessary to pull the tube 60 from the outer unit 101. Therefore, the risk of an operating mistake is reduced.

Moreover, since the stopper is not used, the number of components constituting the indwelling needle device 100 is reduced, and thus the cost of the indwelling needle device 100 can be reduced.

Furthermore, since the inner needle 50 and the outer needle 30 are fastened to each other when the inner unit 102 is at the initial position, after the indwelling needle device 100 is shipped with the inner unit 102 being at the initial position, displacement of the inner unit 102 from the initial position due to vibrations or the like during transportation does not occur.

### Embodiment 2

According to Embodiment 1 described above, the small diameter region 33 of the outer needle 30 and the inner needle 50 are fastened to each other so that the inner unit 102 located at the initial position is prevented from moving toward the retracted position due to the reaction force exerted on the inner needle 50 during insertion. According to Embodiment 2, the outer hub 25 and the inner hub 40 are fastened to each other instead. Hereinafter, an indwelling needle device according to Embodiment 2 will be described with a focus on differences from Embodiment 1.

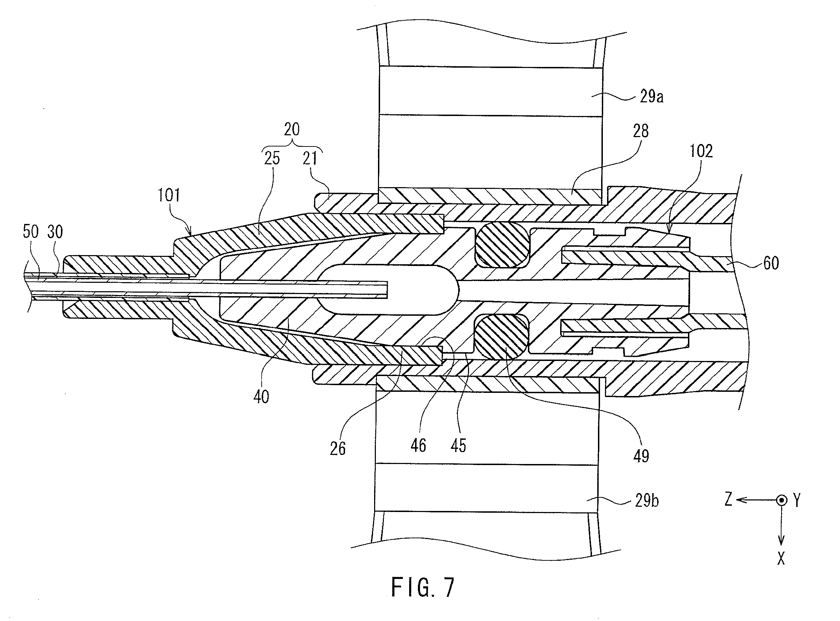

FIG. 7 is a cross-sectional view showing the outer hub 25 and the inner hub 40, and the vicinity thereof, of the indwelling needle device according to Embodiment 2 when the inner unit 102 is at the initial position and taken along a horizontal plane (XZ plane). As shown in FIG. 7, when the inner unit 102 is at the initial position, a front edge of the large diameter portion 45 of the inner hub 40 abuts against a rear edge of the outer hub 25, and thus the inner unit 102 is positioned relative to the outer unit 101 in the front-rear direction (Z axis direction).

At this time, the small diameter portion 46 of the inner hub 40 and the inner circumferential face 26 of the outer hub 25 oppose each other. In Embodiment 2, the inner diameter of the inner circumferential face 26 of the outer hub 25 is equal to or slightly smaller than the outer diameter of the small diameter portion 46 of the inner hub 40. Accordingly, as shown in FIG. 7, when the inner unit 102 is disposed at the initial position, the small diameter portion 46 of the inner hub 40 is fitted into and comes into close contact with the inner circumferential face 26 of the outer hub 25. In the case where the inner diameter of the inner circumferential face 26 of the outer hub 25 is smaller than the outer diameter of the small diameter portion 46 of the inner hub 40, at least one of the inner circumferential face 26 of the outer hub 25 and the small diameter portion 46 of the inner hub 40 is deformed to such an extent that the small diameter portion 46 of the inner hub 40 can be fitted into the inner circumferential face 26 of the outer hub 25. The friction between the small diameter portion 46 and the inner circumferential face 26 is large, causing the outer hub 25 and the inner hub 40 to be fastened to each other. To move the inner unit 102 located at the initial position to the retracted position, a large force that is enough to counteract the fastening force between the outer hub 25 and the inner hub 40 is required.

A method for using the indwelling needle device of Embodiment 2 is generally the same as that of Embodiment 1.

In the insertion operation, the inner needle 50 and the outer needle 30 are inserted into a blood vessel of the patient in a state in which the inner unit 102 is at the initial position and the inner needle 50 protrudes from the leading end of the outer needle 30 (see FIGS. 1 and 2). At this time, a reaction force in a direction in which the inner unit 102 is moved to the retracted position side relative to the outer unit 101 is exerted on the inner needle 50. However, as described above, since the inner circumferential face 26 of the outer hub 25 and the small diameter portion 46 of the inner hub 40 are fastened to each other, the inner unit 102 does not move from the initial position relative to the outer unit 101.

In the subsequent retraction operation, the inner unit 102 is retracted by pulling the tube 60 in a state in which the outer needle 30 is inserted in the patient. It is necessary to apply a large tensile force to the tube 60 in order to pull the small diameter portion 46 of the inner hub 40 out of the inner circumferential face 26 of the outer hub 25. Afterward, when the small diameter portion 46 of the inner hub 40 has come out of the inner circumferential face 26 of the outer hub 25, the tube 60 can be pulled with a relatively small tensile force. The inner unit 102 is moved to the retracted position (see FIGS. 4 and 5) by pulling the tube 60.

As described above, according to the indwelling needle device of Embodiment 2, when the inner unit 102 is at the initial position, the inner circumferential face 26 of the outer hub 25 and the small diameter portion 46 of the inner hub 40 are fastened to each other. Accordingly, similarly to Embodiment 1, even when a reaction force is exerted on the inner needle 50 during the insertion operation, the inner unit 102 does not move toward the retracted position and remains at the initial position. Therefore, the stopper 970 (FIG. 14), which is essential for the conventional indwelling needle device 900 (FIGS. 12 to 15), is no longer necessary in the indwelling needle device of Embodiment 2.

Since the stopper is unnecessary, the risk of an operating mistake that may be made with respect to the conventional indwelling needle device 900 provided with the stopper 970 is reduced. Moreover, the number of components constituting the indwelling needle device is reduced, and thus the cost of the indwelling needle device can be reduced. Furthermore, displacement of the inner unit 102 from the initial position due to vibrations or the like during transportation does not occur.

In the example described above, when the inner unit 102 is at the initial position, the inner circumferential face 26 of the outer hub 25 and the small diameter portion 46 of the inner hub 40 are fastened to each other. However, those portions of the outer hub 25 and the inner hub 40 that are fastened to each other are not limited to this example, and may be other portions.

Embodiment 2 is the same as Embodiment 1 except for the above-described differences.

The configuration shown in Embodiment 2, in which the outer hub 25 and the inner hub 40 are fastened to each other, can be combined with Embodiment 1.

### Embodiment 3

According to Embodiment 1 described above, the small diameter region 33 of the outer needle 30 and the inner needle 50 are fastened to each other so as to prevent the inner unit 102 located at the initial position from moving toward the retracted position due to the reaction force exerted on the inner needle 50 during insertion. According to Embodiment 3, the shield tube 21 and the inner hub 40 are fastened to each other instead. Hereinafter, an indwelling needle device according to Embodiment 3 will be described with a focus on differences from Embodiment 1.

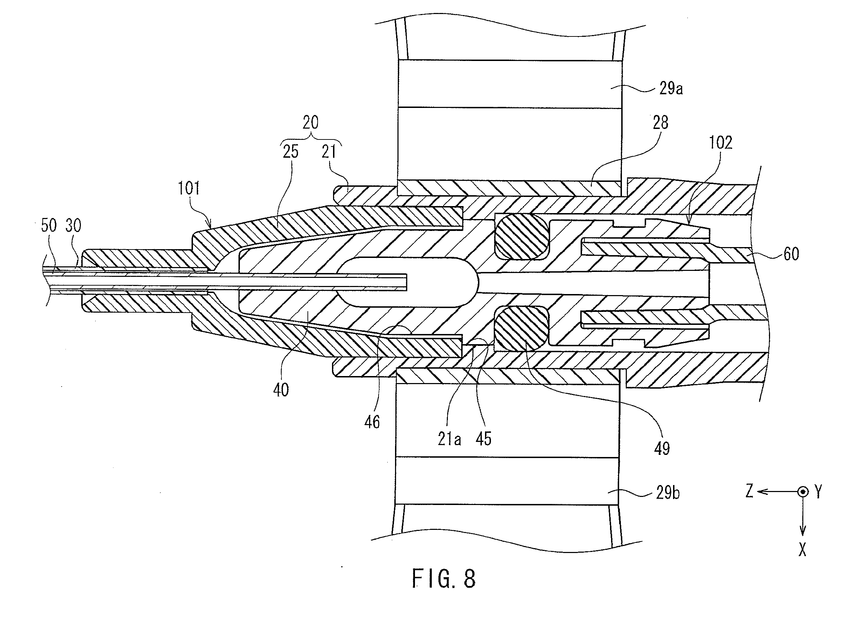

FIG. 8 is a cross-sectional view showing the inner hub 40, and the vicinity thereof, of the indwelling needle device according to Embodiment 3 when the inner unit 102 is at the initial position and taken along a horizontal plane (XZ plane). As shown in FIG. 8, when the inner unit 102 is at the initial position, the front edge of the large diameter portion 45 of the inner hub 40 abuts against the rear edge of the outer hub 25, and thus the inner unit 102 is positioned relative to the outer unit 101 in the front-rear direction (Z axis direction).

According to Embodiment 3, in a region of the inner circumferential face of the shield tube 21 that is adjacent to the outer hub 25, a small diameter portion 21a having a smaller inner diameter than a region rearward of that small diameter portion 21a is formed. When the inner unit 102 is at the initial position, the small diameter portion 21a opposes the large diameter portion 45 of the inner hub 40. The inner diameter of the small diameter portion 21a of the shield tube 21 is equal to or slightly smaller than the outer diameter of the large diameter portion 45 of the inner hub 40. Accordingly, as shown in FIG. 8, when the inner unit 102 is disposed at the initial position, the large diameter portion 45 of the inner hub 40 is fitted into and comes into close contact with the small diameter portion 21a of the shield tube 21. In the case where the inner diameter of the small diameter portion 21a of the shield tube 21 is smaller than the outer diameter of the large diameter portion 45 of the inner hub 40, at least one of the small diameter portion 21a of the shield tube 21 and the large diameter portion 45 of the inner hub 40 is deformed to such an extent that the large diameter portion 45 of the inner hub 40 can be fitted into the small diameter portion 21a of the shield tube 21. The friction between the large diameter portion 45 and the small diameter portion 21a is large, causing the shield tube 21 and the inner hub 40 to be fastened to each other. To move the inner unit 102 located at the initial position to the retracted position, a large force that is enough to counteract the fastening force between the shield tube 21 and the inner hub 40 is required.

A method for using the indwelling needle device according to Embodiment 3 is generally the same as that of Embodiment 1.

In the insertion operation, the inner needle 50 and the outer needle 30 are inserted into a blood vessel of the patient in a state in which the inner unit 102 is at the initial position and the inner needle 50 protrudes from the leading end of the outer needle 30 (see FIGS. 1 and 2). At this time, a reaction force in a direction in which the inner unit 102 is moved to the retracted position side relative to the outer unit 101 is exerted on the inner needle 50. However, since the small diameter portion 21a of the shield tube 21 and the large diameter portion 45 of the inner hub 40 are fastened to each other as described above, the inner unit 102 does not move from the initial position relative to the outer unit 101.

In the subsequent retraction operation, the inner unit 102 is retracted by pulling the tube 60 in a state in which the outer needle 30 is inserted in the patient. In order to pull the large diameter portion 45 of the inner hub 40 out of the small diameter portion 21a of the shield tube 21, it is necessary to apply a large tensile force to the tube 60. Afterward, when the large diameter portion 45 of the inner hub 40 has come out of the small diameter portion 21b of the shield tube 21, the tube 60 can be pulled with a relatively small tensile force. The inner unit 102 is moved to the retracted position (see FIGS. 4 and 5) by pulling the tube 60.

As described above, in the indwelling needle device according to Embodiment 3, when the inner unit 102 is at the initial position, the small diameter portion 21a of the shield tube 21 and the large diameter portion 45 of the inner hub 40 are fastened to each other. Accordingly, similarly to Embodiment 1, even when a reaction force is exerted on the inner needle 50 during the insertion operation, the inner unit 102 does not move toward the retracted position and remains at the initial position. Therefore, the stopper 970 (FIG. 14), which is essential for the conventional indwelling needle device 900 (FIGS. 12 to 15), is no longer necessary in the indwelling needle device according to Embodiment 3.

Since the stopper is unnecessary, the risk of an operating mistake that may be made with respect to the conventional indwelling needle device 900 provided with the stopper 970 is reduced. Moreover, the number of components constituting the indwelling needle device is reduced, and thus the cost of the indwelling needle device can be reduced. Furthermore, displacement of the inner unit 102 from the initial position due to vibrations or the like during transportation does not occur.

In the above-described example, when the inner unit 102 is at the initial position, the large diameter portion 45 of the inner hub 40 is fastened to the shield tube 21. However, the portion of the inner hub 40 that is fastened to the shield tube 21 is not limited to this portion and may be other portions.

Embodiment 3 is the same as Embodiment 1 except for the above-described differences.

The configuration shown in Embodiment 3, in which the shield tube 21 and the inner hub 40 are fastened to each other, can be combined with Embodiments 1 and 2.

### Embodiment 4

According to Embodiment 1 described above, the small diameter region 33 of the outer needle 30 and the inner needle 50 are fastened to each other so as to prevent the inner unit 102 located at the initial position from moving toward the retracted position due to the reaction force exerted on the inner needle 50 during insertion. According to Embodiment 4, the shield tube 21 and the O-ring 49 are fastened to each other instead. Hereinafter, an indwelling needle device according to Embodiment 4 will be described with a focus on differences from Embodiment 1.

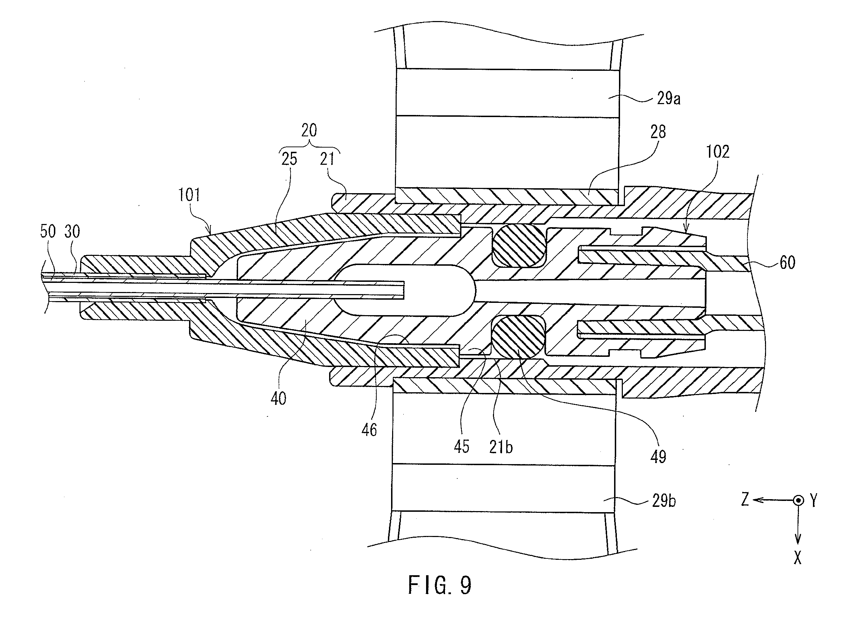

FIG. 9 is a cross-sectional view showing the inner hub 40, and the vicinity thereof, of the indwelling needle device according to Embodiment 4 when the inner unit 102 is at the initial position and taken along a horizontal plane (XZ plane). As shown in FIG. 9, when the inner unit 102 is at the initial position, the front edge of the large diameter portion 45 of the inner hub 40 abuts against the rear edge of the outer hub 25, and thus the inner unit 102 is positioned relative to the outer unit 101 in the front-rear direction (Z axis direction).

According to Embodiment 4, in a region of the inner circumferential face of the shield tube 21 that is adjacent to the outer hub 25, a small diameter portion 21b having a smaller inner diameter than a region rearward of that small diameter portion 21b is formed. When the inner unit 102 is at the initial position, the small diameter portion 21b opposes the O-ring 49 that is attached to the inner hub 40. Accordingly, as shown in FIG. 9, when the inner unit 102 is disposed at the initial position, the O-ring 49 is fitted into the small diameter portion 21b of the shield tube 21, and the O-ring 49 is squeezed in the radial direction and comes into close contact with the inner face of the small diameter portion 21b. The friction between the O-ring 49 and the small diameter portion 21b is large, causing the shield tube 21 and the O-ring 49 to be fastened to each other. To move the inner unit 102 located at the initial position to the retracted position, a large force that is enough to counteract the fastening force between the shield tube 21 and the O-ring 49 is required. It should be noted that unlike the small diameter portion 21a described in Embodiment 3, the small diameter portion 21b does not come into contact with the large diameter portion 45 of the inner hub 40.

A method for using the indwelling needle device according to Embodiment 4 is generally the same as that of Embodiment 1.

In the insertion operation, the inner needle 50 and the outer needle 30 are inserted into a blood vessel of the patient in a state in which the inner unit 102 is at the initial position and the inner needle 50 protrudes from the leading end of the outer needle 30 (see FIGS. 1 and 2). At this time, a reaction force in a direction in which the inner unit 102 is moved to the retracted position side relative to the outer unit 101 is exerted on the inner needle 50. However, since the small diameter portion 21b of the shield tube 21 and the O-ring 49 attached to the inner hub 40 are fastened to each other as described above, the inner unit 102 does not move from the initial position relative to the outer unit 101.

In the subsequent retraction operation, the inner unit 102 is retracted by pulling the tube 60 in a state in which the outer needle 30 is inserted in the patient. In order to pull the O-ring 49 out of the small diameter portion 21b of the shield tube 21, it is necessary to apply a large tensile force to the tube 60. Afterward, when the O-ring 49 has come out of the small diameter portion 21b of the shield tube 21, the tube 60 can be pulled with a relatively small tensile force. After the O-ring 49 is moved rearward of the small diameter portion 21b, liquid-tightness between the O-ring 49 and the shield tube 21 is still secured. The inner unit 102 is moved to the retracted position (see FIGS. 4 and 5) by pulling the tube 60.

As described above, in the indwelling needle device according to Embodiment 4, when the inner unit 102 is at the initial position, the small diameter portion 21b of the shield tube 21 and the O-ring 49 attached to the inner hub 40 are fastened to each other. Accordingly, similarly to Embodiment 1, even when a reaction force is exerted on the inner needle 50 during the insertion operation, the inner unit 102 does not move toward the retracted position and remains at the initial position. Therefore, the stopper 970 (FIG. 14), which is essential for the conventional indwelling needle device 900 (FIGS. 12 to 15), is no longer necessary in the indwelling needle device according to Embodiment 4.

Since the stopper is unnecessary, the risk of an operating mistake that may be made with respect to the conventional indwelling needle device 900 provided with the stopper 970 is reduced. Moreover, the number of components constituting the indwelling needle device is reduced, and thus the cost of the indwelling needle device can be reduced. Furthermore, displacement of the inner unit 102 from the initial position due to vibrations or the like during transportation does not occur.

Embodiment 4 is the same as Embodiment 1 except for the above-described differences.

The configuration shown in Embodiment 4, in which the shield tube 21 and the O-ring 49 are fastened to each other, can be combined with Embodiments 1 to 3.

### Embodiment 5

According to Embodiment 1 described above, the small diameter region 33 of the outer needle 30 and the inner needle 50 are fastened to each other so as to prevent the inner unit 102 located at the initial position from moving toward the retracted position due to the reaction force exerted on the inner needle 50 during insertion. According to Embodiment 5, the outer hub 25 and the O-ring 49 are fastened to each other instead. Hereinafter, an indwelling needle device according to Embodiment 5 will be described with a focus on differences from Embodiment 1.

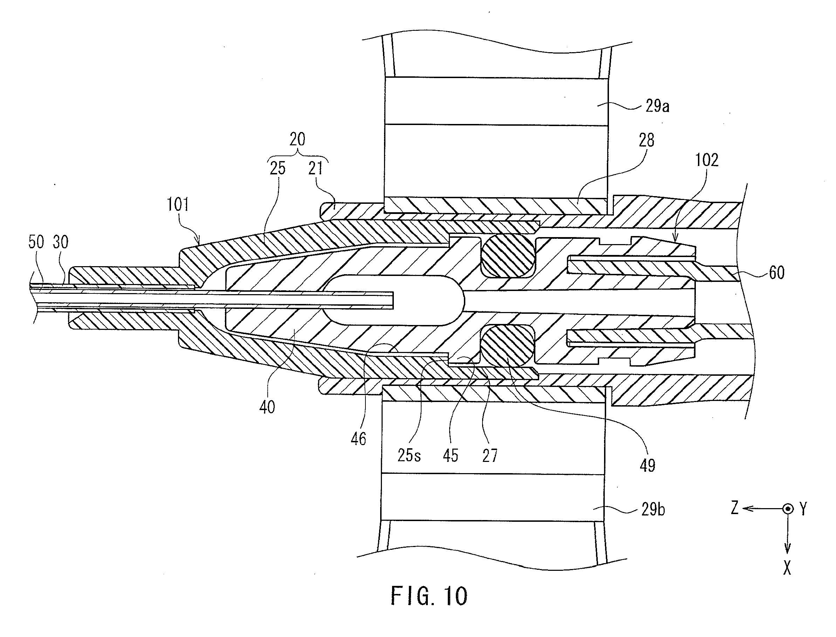

FIG. 10 is a cross-sectional view showing the inner hub 40, and the vicinity thereof, of the indwelling needle device according to Embodiment 5 when the inner unit 102 is at the initial position and taken along a horizontal plane (XZ plane). According to Embodiment 5, the outer hub 25 is extended rearward so that, when the inner unit 102 is at the initial position, the outer hub 25 opposes the O-ring 49 attached to the inner hub 40. A step 25s is formed on the inner circumferential face of the outer hub 25 by changing the inner diameter of the outer hub 25. A region of the outer hub 25 that is rearward of the step 25s constitutes an extended portion 27 having a larger inner diameter than a region that is forward of the step 25s. The inner diameter of the extended portion 27 is smaller than the inner diameter of a region of the shield tube 21 that is rearward of the extended portion 27.

As shown in FIG. 10, when the inner unit 102 is at the initial position, the front edge of the large diameter portion 45 of the inner hub 40 abuts against the step 25s that is formed on the inner circumferential face of the outer hub 25, and thus the inner unit 102 is positioned relative to the outer unit 101 in the front-rear direction (Z axis direction). Also, when the inner unit 102 is at the initial position, the O-ring 49 attached to the inner hub 40 opposes the extended portion 27 of the outer hub 25. Accordingly, as shown in FIG. 10, when the inner unit 102 is disposed at the initial position, the O-ring 49 is fitted into the extended portion 27 of the outer hub 25, and the O-ring 49 is squeezed in the radial direction and comes into close contact with an inner face of the extended portion 27. The friction between the O-ring 49 and the extended portion 27 is large, causing the outer hub 25 and the O-ring 49 to be fastened to each other. To move the inner unit 102 located at the initial position to the retracted position, a large force that is enough to counteract the fastening force between the outer hub 25 and the O-ring 49 is required. It should be noted that unlike the small diameter portion 21a of the shield tube 21 described in Embodiment 3, the extended portion 27 does not come into contact with the large diameter portion 45 of the inner hub 40.

A method for using the indwelling needle device according to Embodiment 5 is generally the same as that of Embodiment 1.

In the insertion operation, the inner needle 50 and the outer needle 30 are inserted into a blood vessel of the patient in a state in which inner unit 102 is at the initial position and the inner needle 50 protrudes from the leading end of the outer needle 30 (see FIGS. 1 and 2). At this time, a reaction force in a direction in which the inner unit 102 is moved to the retracted position side relative to the outer unit 101 is exerted on the inner needle 50. However, since the extended portion 27 of the outer hub 25 and the O-ring 49 attached to the inner hub 40 are fastened to each other as described above, the inner unit 102 does not move from the initial position relative to the outer unit 101.

In the subsequent retraction operation, the inner unit 102 is retracted by pulling the tube 60 in a state in which the outer needle 30 is inserted in the patient. In order to pull the O-ring 49 out of the extended portion 27 of the outer hub 25, it is necessary to apply a large tensile force to the tube 60. Afterward, when the O-ring 49 has come out of the extended portion 27 of the outer hub 25, the tube 60 can be pulled with a relatively small tensile force. After the O-ring 49 is moved rearward of the extended portion 27, the liquid-tightness between the O-ring 49 and the shield tube 21 is still secured. The inner unit 102 is moved to the retracted position (see FIGS. 4 and 5) by pulling the tube 60.

As described above, in the indwelling needle device according to Embodiment 5, when the inner unit 102 is at the initial position, the extended portion 27 of the outer hub 25 and the O-ring 49 attached to the inner hub 40 are fastened to each other. Accordingly, similarly to Embodiment 1, even when a reaction force is exerted on the inner needle 50 during the insertion operation, the inner unit 102 does not move toward the retracted position and remains at the initial position. Therefore, the stopper 970 (FIG. 14), which is essential for the conventional indwelling needle device 900 (FIGS. 12 to 15), is no longer necessary in the indwelling needle device according to Embodiment 5.

Since the stopper is unnecessary, the risk of an operating mistake that may be made with respect to the conventional indwelling needle device 900 provided with the stopper 970 is reduced. Moreover, the number of components constituting the indwelling needle device is reduced, and thus the cost of the indwelling needle device can be reduced. Furthermore, displacement of the inner unit 102 from the initial position due to vibrations or the like during transportation does not occur.

Embodiment 5 is the same as Embodiment 1 except for the above-described differences.

The configuration shown in Embodiment 5, in which the outer hub 25 and the O-ring 49 are fastened to each other, can be combined with Embodiments 1 to 3.

### Embodiment 6

According to Embodiment 1 described above, the small diameter region 33 of the outer needle 30 and the inner needle 50 are fastened to each other so as to prevent the inner unit 102 located at the initial position from moving toward the retracted position due to the reaction force exerted on the inner needle 50 during insertion. According to Embodiment 6, the outer hub 25 and the inner needle 50 are fastened to each other instead. Hereinafter, an indwelling needle device according to Embodiment 6 will be described with a focus on differences from Embodiment 1.

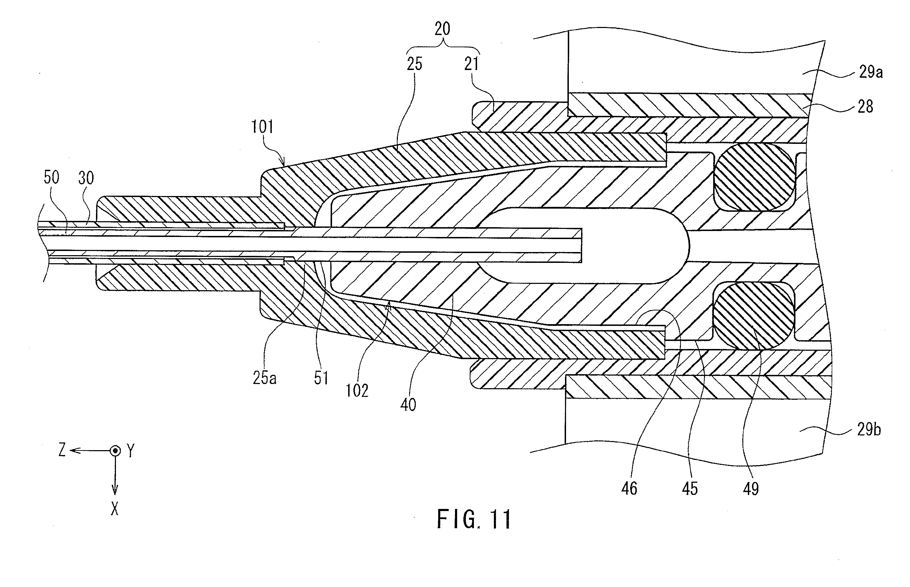

FIG. 11 is a cross-sectional view showing the outer hub 25, and the vicinity thereof, of the indwelling needle device according to Embodiment 6 when the inner unit 102 is at the initial position and taken along a horizontal plane (XZ plane). As shown in FIG. 11, when the inner unit 102 is at the initial position, the front edge of the large diameter portion 45 of the inner hub 40 abuts against the rear edge of the outer hub 25, and thus the inner unit 102 is positioned relative to the outer unit 101 in the front-rear direction (Z axis direction).

According to Embodiment 6, a large diameter portion 51 having a relatively large outer diameter is formed on the outer face of the inner needle 50. The large diameter portion 51 is formed in a region of the inner needle 50 that is located closer to the inner hub 40 than the outer needle 30 is when the inner unit 102 is at the initial position. The outer diameter of the large diameter portion 51 of the inner needle 50 is larger than the outer diameter of a region of the inner needle 50 that is forward of the large diameter portion 51. On the other hand, a small diameter portion 25a having a relatively small inner diameter is formed in the outer hub 25 in order to position the outer needle 30 in the front-rear direction, the small diameter portion 25a being adjacent to a portion of the outer hub 25 that holds the outer needle 30. When the inner unit 102 is at the initial position, the small diameter portion 25a opposes the large diameter portion 51 of the inner needle 50. The inner diameter of the small diameter portion 25a of the outer hub 25 is equal to or slightly smaller than the outer diameter of the large diameter portion 51 of the inner needle 50. Accordingly, as shown in FIG. 11, when the inner unit 102 is disposed at the initial position, the large diameter portion 51 of the inner needle 50 is fitted into and comes into close contact with the small diameter portion 25a of the outer hub 25. In the case where the inner diameter of the small diameter portion 25a of the outer hub 25 is smaller than the outer diameter of the large diameter portion 51 of the inner needle 50, at least one of the small diameter portion 25a of the outer hub 25 and the large diameter portion 51 of the inner needle 50 is deformed to such an extent that the large diameter portion 51 of the inner needle 50 can be fitted into the small diameter portion 25a of the outer hub 25. The friction between the large diameter portion 51 and the small diameter portion 25a is large, causing the outer hub 25 and the inner needle 50 to be fastened to each other. To move the inner unit 102 located at the initial position to the retracted position, a large force that is enough to counteract the fastening force between the outer hub 25 and the inner needle 50 is required.

A method for using the indwelling needle device according to Embodiment 6 is generally the same as that of Embodiment 1.

In the insertion operation, the inner needle 50 and the outer needle 30 are inserted into a blood vessel of the patient in a state in which the inner unit 102 is at the initial position and the inner needle 50 protrudes from the leading end of the outer needle 30 (see FIGS. 1 and 2). At this time, a reaction force in a direction in which the inner unit 102 is moved to the retracted position side relative to the outer unit 101 is exerted on the inner needle 50. However, since the small diameter portion 25a of the outer hub 25 and the large diameter portion 51 of the inner needle 50 are fastened to each other as described above, the inner unit 102 does not move from the initial position relative to the outer unit 101.

In the subsequent retraction operation, the inner unit 102 is retracted by pulling the tube 60 in a state in which the outer needle 30 is inserted in the patient. In order to pull the large diameter portion 51 of the inner needle 50 out of the small diameter portion 25a of the outer hub 25, it is necessary to apply a large tensile force to the tube 60. Afterward, when the large diameter portion 51 of the inner needle 50 has come out of the small diameter portion 25a of the outer hub 25, the tube 60 can be pulled with a relatively small tensile force. The inner unit 102 is moved to the retracted position (see FIGS. 4 and 5) by pulling the tube 60.

As described above, in the indwelling needle device according to Embodiment 6, when the inner unit 102 is at the initial position, the small diameter portion 25a of the outer hub 25 and the large diameter portion 51 of the inner needle 50 are fastened to each other. Accordingly, similarly to Embodiment 1, even when a reaction force is exerted on the inner needle 50 during the insertion operation, the inner unit 102 does not move toward the retracted position and remains at the initial position. Therefore, the stopper 970 (FIG. 14), which is essential for the conventional indwelling needle device 900 (FIGS. 12 to15), is no longer necessary in the indwelling needle device of the Embodiment 6.

Since the stopper is unnecessary, the risk of an operating mistake that may be made with respect to the conventional indwelling needle device 900 provided with the stopper 970 is reduced. Moreover, the number of components constituting the indwelling needle device is reduced, and thus the cost of the indwelling needle device can be reduced. Furthermore, displacement of the inner unit 102 from the initial position due to vibrations or the like during transportation does not occur.

In the above-described example, the large diameter portion 51 is formed in the inner needle 50, and this large diameter portion 51 and the small diameter portion 25a of the outer hub 25 are fastened to each other. However, it is also possible to set the outer diameter of the inner needle 50 to be entirely constant in the longitudinal direction and set the inner diameter of the small diameter portion 25a of the outer hub 25 such that the small diameter portion 25a is fastened to the outer circumferential face of the inner needle 50. In this case, in the retraction operation, it is necessary to pull the tube 60 with a relatively large force until the inner needle 50 comes out of the small diameter portion 25a.

Embodiment 6 is the same as Embodiment 1 except for the above-described differences.

The configuration shown in Embodiment 6, in which the outer hub 25 and the inner needle 50 are fastened to each other, can be combined with Embodiments 1 to 5.

Embodiments 1 to 6 described above should be considered as illustrative only. The present invention is not limited to Embodiments 1 to 6 described above, and can be modified as appropriate.

Those portions of the inner unit 102 and the outer unit 101 that are fastened to each other when the inner unit 102 is at the initial position are not limited to the examples shown in Embodiments 1 to 6. Portions of the inner unit 102 and the outer unit 101 other than those shown in Embodiments 1 to 6 may also be fastened to each other.

The fastening force between the inner unit 102 and the outer unit 101 can be set taking the reaction force that is exerted on the inner needle 50 during the insertion operation, the force that is required to move the inner unit 102 during the retraction operation, and the like into account. That is to say, the fastening force between the inner unit 102 and the outer unit 101 is set to be larger than the reaction force exerted on the inner needle 50 during the insertion operation so that the inner unit 102 is prevented from moving relative to the outer unit 101 due to that reaction force. However, the upper limit of the fastening force is set such that the retraction operation that moves the inner unit 102 located at the initial position to the retracted position can be performed.

Instead of the configuration in which a single portion of the inner unit 102 and a single portion of the outer unit 101 are fastened to each other when the inner unit 102 is at the initial position, it is also possible that a plurality of portions of the inner unit 102 and a plurality of portions of the outer unit 101 are fastened to each other. For example, a configuration may also be adopted in which at least two portions of the inner unit 102 and at least two portions of the outer unit 101 that are selected as desired from the fastening portions of the inner unit 102 and the outer unit 101 shown in Embodiments 1 to 6 are fastened to each other.

Fastening between the inner unit 102 and the outer unit 101 of the present invention is caused by the friction between the inner unit 102 and the outer unit 101. It is also possible to keep the inner unit 102 at the initial position by combining other methods such as engagement between the inner unit 102 and the outer unit 101 with the fastening between the inner unit 102 and the outer unit 101.

The configurations of the outer unit 101 and the inner unit 102 can be modified as desired without departing from the scope of the present invention.

For example, a large-area flat surface portion may be provided on the outer face of the shield 20 so that the indwelling needle device can be easily gripped, or an uneven shape for preventing slippage may be formed thereon.

The structure for fitting the hub 40 located at the retracted position and the shield 20 to each other may also have a configuration other than the above-described configuration. Alternatively, the fitting structure may be omitted.

The outer unit 101 shown in Embodiments 1 to 6 above includes the shield tube 21, the outer hub 25, and the outer needle 30 that are separate components. However, the shield tube 21 and the outer hub 25 may be integrated and configured as a single component, and furthermore, the shield tube 21, the outer hub 25, and the outer needle 30 may be integrated and configured as a single component. The wings 29a and 29b and the fixing member 28 that are attached to the outer unit 101 may be omitted.

In Embodiments 1 to 6 described above, the inner unit 102 is positioned at the initial position relative to the outer unit 101 by abutting the front edge of the large diameter portion 45 of the inner hub 40 against the rear edge of the outer hub 25 (Embodiments 1 to 4 and 6) or the step 25s of the inner circumferential face of the outer hub 25 (Embodiment 5). However, it is also possible to regulate the position of the inner unit 102 in the front-rear direction when the inner unit 102 is located at the initial position by bringing portions of the inner unit 102 and the outer unit 101 other than the above-described portions into contact with each other.

In the foregoing description, the indwelling needle device of the present invention is used for hemodialysis. However, the application of the indwelling needle device of the present invention is not limited to hemodialysis, and the indwelling needle device of the present invention can be used in any application, such as infusion and blood transfusion, that uses an indwelling needle device.

### Industrial Applicability

There is no particular limitation on the field of use of the present invention, and the present invention can be extensively used as an indwelling needle device for use in such treatments as infusion, blood transfusion, extracorporeal blood circulation, and the like. Among these, the present invention can be preferably used as an indwelling needle device for hemodialysis.

### List of Reference Numerals

- 100: Indwelling needle device
- 101: Outer unit
- 102: Inner unit
- 20: Shield
- 21: Shield tube
- 24: Inner cavity of shield
- 25: Outer hub
- 30: Outer needle
- 40: Inner hub
- 49: O-ring
- 50: Inner needle
- 60: Tube

## Claims

1. An indwelling needle device, comprising:
an outer unit including a shield that has an inner cavity and a soft outer needle that is fixed to a front end of the shield; and
an inner unit including an inner hub that is disposed within the inner cavity of the shield, a hard inner needle that is fixed to a front end of the inner hub, and a tube that is connected to a rear end of the inner hub,
the inner unit being displaceable relative to the outer unit from an initial position at which the inner needle penetrates the outer needle and protrudes from a leading end of the outer needle to a retracted position at which the inner needle is housed within the inner cavity of the shield,
wherein the inner unit located at the initial position is fastened to the outer unit so that the inner unit is prevented from moving toward the retracted position due to a reaction force that is exerted on the inner needle during insertion with the inner unit being disposed at the initial position.

2. The indwelling needle device according to claim 1,
wherein when the inner unit is at the initial position, the inner needle is fastened to the outer needle.

3. The indwelling needle device according to claim 1 or 2,
wherein when the inner unit is at the initial position, the inner hub is fastened to the shield.

4. The indwelling needle device according to claim 3,
wherein the shield includes an outer hub that holds the outer needle and a tube-shaped shield tube, the outer hub being fixed to an end of the shield tube, and
when the inner unit is at the initial position, the inner hub is fastened to the outer hub.

5. The indwelling needle device according to claim 3 or 4,
wherein the shield includes an outer hub that holds the outer needle and a tube-shaped shield tube, the outer hub being fixed to an end of the shield tube, and
when the inner unit is at the initial position, the inner hub is fastened to the shield tube.

6. The indwelling needle device according to any one of claims 1 to 5,
wherein when the inner unit is at the initial position, an O-ring attached to the inner hub is fastened to the shield.

7. The indwelling needle device according to claim 6,
wherein the shield includes an outer hub that holds the outer needle and a tube-shaped shield tube, the outer hub being fixed to an end of the shield tube, and
when the inner unit is at the initial position, the O-ring is fastened to the shield tube.

8. The indwelling needle device according to claim 6,
wherein the shield includes an outer hub that holds the outer needle and a tube-shaped shield tube, the outer hub being fixed to an end of the shield tube, and
when the inner unit is at the initial position, the O-ring is fastened to the outer hub.

9. The indwelling needle device according to any one of claims 1 to 8,
wherein when the inner unit is at the initial position, the inner needle is fastened to the shield.

10. The indwelling needle device according to claim 9,
wherein the shield includes an outer hub that holds the outer needle and a tube-shaped shield tube, the outer hub being fixed to an end of the shield tube, and
when the inner unit is at the initial position, the inner needle is fastened to the outer hub.

11. The indwelling needle device according to any one of claims 1 to 10,
wherein the fastening is caused by friction between the inner unit and the outer unit.
